# EUROPÄISCHE PATENTSCHRIFT

(11) **EP 0 702 232 B1**
(45) Veröffentlichungstag und Bekanntmachung des Hinweises auf die Patenterteilung: **08.11.2000**
(21) Anmeldenummer: 95111663.1
(22) Anmeldetag: 25.07.1995
(51) Int. Cl.: G01N 33/52

(54) **Verfahren zur Untersuchung des Zustandes von menschlichem Haar**
Process for evaluating the state of human hair
Procédé pour évaluer l'état des cheveux humains

(30) Priorität: 15.09.1994 DE 4432854
(43) Veröffentlichungstag der Anmeldung: 20.03.1996
(73) Patentinhaber: GOLDWELL GmbH, D-64297 Darmstadt (DE)
(72) Erfinder: Morita, Kenichi, D-64625 Bensheim (DE)

(56) Entgegenhaltungen:
- EP-A- 0 193 692
- GB-A- 2 027 195
- CHEMICAL ABSTRACTS, Band 105, Nr. 8, 25. August 1986, Columbus, Ohio, USA G. BLANKENBURG et al. "Determination of the dye distribution in human hair cross sections using a scanning photometer microscope" Seite 361, Nr. 66 233w; & J.Soc.Cosmet.Chem. 1986, 37(2), 59-71
- CHEMICAL ABSTRACTS, Band 80, Nr. 14, 8. April 1974, Columbus, Ohio, USA H.P. KUBERSKY "Tests on the adsorption of active agents on human hair" Seite 236, Nr. 74 249m; & Parfuem. Kosmet. 1973, 54(12), 371-6

## Beschreibung

Die Erfindung betrifft ein Verfahren zur Untersuchung des Zustandes von menschlichen Haaren, insbesondere die Bestimmung einer eventuellen Schädigung, und die Verwendung eines solchen Mittels zur visuellen Bestimmung des Schädigungsgrades.

Es besteht ein Bedürfnis nach einfach durchzuführenden Bestimmungsverfahren für den Zustand menschlicher Haare, insbesondere zur Ermittlung einer eventuellen Schädigung der Keratinstruktur durch Umwelteinflüsse, wiederholte Dauerwellung, Oxidationsfärbung oder Aufhellung.

Die Ermittlung einer eventuellen Schädigung ist besonders wichtig, um aus deren Ausmaß auf den notwendigen Umfang von Gegenmaßnahmen schließen zu können, d.h., die Anwendung geeigneter Konditionier- und Restrukturierungsmittel.

Aus der EP-A 193 692 ist bereits ein Verfahren zur Bestimmung des Oberflächenzustands von Keratinfasern, insbesondere menschlichen Haaren, bekannt, das aus einem kombinierten Anfärbungsverfahren einer Haarprobe durch Oxidationsfarbstoffvorprodukte und einem nachfolgenden Extraktionsverfahren des an der Oberfläche der Haarprobe gebundenen Farbstoffs besteht, wobei durch dessen Menge in der Extraktionslösung dann der Schädigungsgrad bestimmt werden kann.

Dieses Verfahren ist relativ umständlich, bedarf eines größeren apparativen Aufwands und ist daher zur Durchführung einer Schnellbestimmung, beispielsweise durch einen Friseur im Rahmen einer Haarbehandlung oder gar durch den Kunden selbst, nicht geeignet.

Es bestand daher ein Bedürfnis, ein einfach und schnell durchführbares Verfahren zur Bestimmung des Zustandes menschlichen Haares und seines eventuellen Schädigungsgrades zur Verfügung zu stellen.

Aus der GB-A-20 27 195 ist bereits ein Verfahren zur Bestimmung der Ionenladung einer Haarprobe bekannt, wobei diese mit einer sauren Lösung mindestens eines sauren oder basischen Farbstoffs behandelt und aus einer eventuellen Farbveränderung auf die Ionogenität der Haarfaseroberfläche geschlossen wird.

Die vorliegende Erfindung löst diese Aufgabe durch die Verwendung einer wäßrigen Lösung, die mindestens einen anionischen Farbstoff und mindestens ein Lösungsmittel, ausgewählt aus der Gruppe C₂-C₆-Alkandiole, deren Ethyl- und Methylethern, Benzylalkohol, Benzyloxyethanol, Phenoxyethanol und/oder Zimtalkohol, enthält, und einen pH-Wert zwischen 1 und 5 aufweist, und in die eine Haarprobe eingebracht wird, und nach einer kurzen Einwirkungszeit, die von der Adsorptionsfähigkeit des eingesetzten Farbstoffs abhängig ist, und vorzugsweise zwischen etwa 15, insbesondere etwa 30 Sekunden und etwa 30 Minuten liegt, durch die Farbveränderung der Lösung der Schädigungsgrad des Haares auf einfache Weise visuell ermittelt wird.

Bevorzugt ist ein pH-Wert der Farbstofflösung zwischen 2 und 3, insbesondere etwa 2,5.

Durch den Zusatz der genannten Lösungsmittel, vorzugsweise in Menge von 1 bis 50 Gew.-%, insbesondere 2,5 bis 25 Gew.-%, bezogen auf die Gesamtzusammensetzung des Mittels, wird die Differenzierung des Haarzustandes über die Färbung verstärkt.

Dadurch wird entweder ein zusätzlicher Quellungs- oder Schrumpfungseffekt auf dem zu untersuchenden Haar erreicht, wodurch die Deutlichkeit der Anfärbung je nach Haarzustand erhöht wird.

Für die Durchführung des Verfahrens ist im Prinzip jeder direktziehende, zugelassene anionische Farbstoff geeignet (d.h., Farbstoff mit Carboxyl- und/oder vorzugsweise Sulfogruppen); es wird hierzu auf die Auflistung in der "Verordnung über kosmetische Mittel" (Kosmetik-Verodnung), Anlage 3, verwiesen.

Besonders bevorzugte Farbstoffe sind in diesem Zusammenhang 1-(4-Sulfo-1-phenylazo)-2-naphthol-6-sulfonsäure bzw. deren Dinatriumsalz (C.I.-No. 15985), der insbesondere zu Bestimmung des Schädigungsgrades bei häufigen Oxidationsbehandlungen unterworfenem Haar geeignet ist, (N-Ethyl-p-sulfobenzylamino)-phenyl-2-sulfophenyl)-methylen-(N-ethyl-N-p-sulfobenzyl)-Δ-2,5-cyclohexadienimin bzw. dessen Dinatriumsalz (C.I.-No. 42090), insbesondere zur Bestimmung des Zustandes von häufig dauergewelltem Haar, 2-(4-Sulfo-1-naphthylazo)-1-naphthtol-4-sulfonsäure bzw. deren Dinatriumsalz und 1-(4'-Sulfophenylazo)-2-hydronaphthtlin bzw. dessen Natriumsalz.

Wie bereits ausgeführt, sind jedoch auch andere direktziehende anionische Farbstoffe und auch Gemische desselben geeignet.

Es handelt sich dabei im Prinzip um die Bestimmung der Säurebindungskapazität der Keratinfasern als ein Maß für die Intaktheit der Oberfläche des Haares.

Die Farbstoffkonzentration in der Lösung ist dabei abhängig von der Adsorptionsfähigkeit des jeweiligen Farbstoffs; sie liegt vorzugsweise zwischen etwa 10⁻³ mM und 10 mM, vorzugsweise zwischen etwa 10⁻² und 10⁻¹ mM.

Geeignete Lösungsmittel sind C₂-C₆-Alkandiole und deren Methyl- und Ethylether wie Ethandiol, 1,2,- und 1,3-Propandiol, 1,2-, 1,3-, 1,4- und 2,3-Butandiol, Diethylenglykol, Dipropylenglykol, 1-Methoxy- und 1-Ethoxypropanol(-2), Diethylenglykolmonoethylether und - monoethylether (Methyl- und Ethyldiglykol), Dipropylenglykolmonomethyl- und -monoethylether sowie Benzylalkohol, Benzyloxyethanol, Phenoxyethanol und/oder Zimtalkohol.

Bevorzugt eingesetzte Lösungsmittel sind Benzylalkohol und Ethyldiglykol; jedoch sind Art und Menge des jeweiligen Lösungsmittels abhängig von Art und Menge des einzusetzenden anionischen Farbstoffs und mit diesem abzustimmen.

Im folgenden werden Beispiele zur erfindungsgemäßen Anwendung der Farbstofflösungen gegeben:

### Beispiel 1

0,1 g einer Haarsträhne, zerkleinert in kleine Teilchen, wurden in 10ml einer wäßrigen Lösung, enthaltend
**6 · 10**^{**-2**}**mM anionischen Farbstoff (C.I.-No. 15985) und**
**3 Gew.-% Benzylalkohol, eingestellt mit Milchsäure auf pH 2,4**
eingebracht.

Nach 15 Minuten zeigte ein deutlicher Farbumschlag nach Dunkelgelb eine auf starke Aufhellung mit Peroxid zurückzuführende Schädigung des Haares an.

Anhand einer Vergleichstabelle läßt sich ermitteln, mit welchem Haarstrukturant eine Behandlung angebracht erscheint.

### Beispiel 2

0,1 g einer in kleine Partikel zerkleinerten Haarsträhne wurden in 10ml einer wäßrigen Lösung, bestehend aus
**4,0 · 10**^{**-2**}**mM anionischen Farbstoff (C.I.-No. 42090),**
**10 Gew.-% Ethyldiglykol, eingestellt mit Milchsäure auf pH 2,4,**
eingebracht.

Nach 20 Minuten zeigte eine deutliche Aufhellung der zuvor dunkelblauen Lösung eine Schädigung des untersuchten Haares durch wiederholte Dauerwellbehandlung an.

Wie in Beispiel 1 beschrieben, läßt sich durch einen Vergleich mit Standard-Farbtabellen ermitteln, welche restruktierende Behandlung angezeigt ist.

Mit dem erfindungsgemäßen Verfahren ist also nicht nur die Ermittlung des Gesamtschädigungsgrades, sondern, durch selektive Auswahl der Farbstoffe und Lösungsmittel, auch eine selektive Bestimmung der Ursachen der Haarschädigung, z.B. durch Aufhellung oder Dauerwellung, möglich.

## Patentansprüche

1. Verfahren zur Untersuchung des Zustandes von menschlichem Haar, wobei eine Haarprobe in eine wäßrige Lösung, die mindestens einen anionischen Farbstoff und mindestens einen pH-Wert zwischen 1 und 5 aufweist und ein Lösungsmittel, ausgewählt aus der Gruppe C₂-C₆-Alkandiole, deren Ethyl- und Methylethern, Benzylalkohol, Benzyloxyethanol, Phenoxyethanol und/oder Zimtalkohol, enthält, eingebracht und anschließend anhand der Farbveränderung dieser Lösung der Schädigungsgrad visuell festgestellt wird.

2. Verfahren nach Anspruch 1, dadurch gekennzeichnet, daß die Haarprobe etwa 30 Sekunden bis etwa 30 Minuten in der Farblösung verbleibt.

3. Verfahren nach Anspruch 1 oder 2, dadurch gekennzeichnet, daß die Farbstofflösung 1 bis 50 Gew.-% mindestens eines Lösungsmittels enthält.

4. Verfahren nach Anspruch 3, dadurch gekennzeichnet, daß die Farbstofflösung Benzylalkohol und/oder Ethyldiglycol enthält.

5. Verfahren nach einem oder mehren der Ansprüche 1 bis 4, dadurch gekennzeichnet, daß die Farbstofflösung einen pH-Wert zwischen 2 und 3 aufweist.

6. Verfahren nach einem oder mehreren der Ansprüche 1 bis 5, dadurch gekennzeichnet, daß die Konzentration des anionischen Farbstoffs in der wäßrigen Lösung 10⁻³ mM bis 10 mM beträgt.

7. Verwendung einer wäßrigen Lösung die mindestens einen anionischen Farbstoff und mindestens ein Lösungsmittel, ausgewählt aus der Gruppe C₂-C₆-Alkandiole, deren Ethyl- und Methylethern, Benzylalkohol, Benzyloxyethanol, Phenoxyethanol und/oder Zimtalkohol, enthält, und einen pH-Wert zwischen 1 und 5 aufweist, zur visuellen Bestimmung des Schädigungsgrades von menschlichen Haaren.

## Claims

1. Process for the determination of the condition of human hair wherein a hair sample is immersed into an aqueous solution of at least one anionic dyestuff having a pH-value between 1 and 5 and comprising at least one solvent, selected from the group of C₂-C₆-alkanediols, the ethyl and methyl ethers thereof, benzyl alcohol, benzyloxyethanol, phenoxyethanol and (or) cinnamyl alcohol, and subsequently the degree of hair damage is visually determined in comparing the color change of the solution.

2. Process according to claim 1, characterized in that the hair sample remains in the color solution from about 30 seconds to about 30 minutes.

3. Process according to claim 1 or 2, characterized in that the dyestuff solution comprises 1% to 50% by wt. of at least one solvent.

4. Process according to claim 3, characterized in that the dyestuff solution comprises benzyl alcohol and (or) ethyl diglycol.

5. Process according to one or more of claims 1 to 4, characterized in that the dyestuff solution has a pH-value between 2 and 3.

6. Process according to one or more of claim 1 to 5, characterized in that the concentration of anionic dyestuff in the aqueous solution is 10⁻³ mM to 10 mM.

7. Use of an aqueous solution of an anionic dyestuff having a pH-value between 1 and 5 and comprising at least one solvent, selected from the group of C₂-C₆-alkanediols, the ethyl and methyl ethers thereof, benzyl alcohol, benzyl oxyethanol, phenoxyerthanol and (or) cinnamyl alcohol for the visual determination of the degree of damage of human hair.

## Revendications

1. Procédé pour l'examen de l'état du cheveau humain, dans lequel un échantillon de cheveu est introduit dans une solution aqueuse d'au moins un colorant anionique, qui présente une valeur de pH comprise entre 1 et 5, et contient au moins un solvant, choisi dans le groupe formé par les alcane-diols en C₂ à C₆, leurs éthers méthyliques et éthyliques, l'alcool benzylique, le benzyloxyéthanol, le phénoxyéthanol et/ou l'alcool cinnamique, puis l'état d'endommagement du cheveu est constaté visuellement à l'aide de la variation de couleur de cette solution.

2. Procédé selon la revendication 1, caractérisé en ce que l'échantillon de cheveu reste dans la solution d'environ 30 secondes à environ 30 minutes.

3. Procédé selon la revendication 1 on 2, caractérisé en ce que la solution de colorant contient 1 à 50% en poids d'au moins un solvant.

4. Procédé selon la revendication 3, caractérisé en ce que la solution de colorant contient de l'alcool benzylique et/ou de l'éthyldiglycol.

5. Procédé selon l'une ou plusieurs des revendications 1 à 4, caractérisé en ce que la solution de colorant présente une valeur de pH comprise entre 2 et 3.

6. Procédé selon l'une ou plusieurs des revendicationds 1 à 5, caractérisé en ce que la concentration du colorant anionique dans la solution aqueuse est de 10⁻³ mM á 10 mM.

7. Utilisation d'une solution aqueuse d'un colorant anionique, qui présente une valeur de pH comprise entre 1 et 5, et contient au mois un solvant, choisi dans le groupe formé par les alcane-diols en C₂ à C₆, leurs éthers méthyliques et éthyliques, l'alcool benzylique, le benzyloxyéthanol, le phénoxyéthanol et/ou l'alcool cinnamique, pour la determination visuelle du degré d'endommagement de cheveux humains.
